# EUROPEAN PATENT SPECIFICATION

(11) **EP 3 058 067 B1**
(45) Date of publication and mention of the grant of the patent: **01.08.2018**
(21) Application number: 14853838.2
(22) Date of filing: 17.10.2014
(51) Int. Cl.: C12N 15/11, C12Q 1/68, C12Q 1/70

(54) **CIRCULATING CANCER BIOMARKER AND ITS USE**
ZIRKULIERENDER KREBSBIOMARKER UND DESSEN VERWENDUNG
BIOMARQUEUR CIRCULANT DU CANCER ET UTILISATION DE CELUI-CI

(30) Priority: 18.10.2013 US 201361892796 P
(43) Date of publication of application: 24.08.2016
(73) Proprietor: National Taiwan University, Taipei 10617 (TW); Chung Wo, Andrew Man, Edgartown, MA 02539 (US)
(72) Inventor: CHEN, Pei-Jer, Taipei 10002 (TW); YEH, Shiou-Hwei, Taipei 10002 (TW); LI, Chiao-Ling, Taipei 10002 (TW); CHEN, Ding-Shinn, Taipei 10002 (TW)
(74) Representative: CMS Cameron McKenna Nabarro Olswang LLP
(86) International application number: PCT/US2014/061127
(87) International publication number: WO 2015/058079

(56) References cited:
- YOSHIKI MURAKAMI ET AL: "Hepatitis B virus DNA in liver, serum, and peripheral blood mononuclear cells after the clearance of serum hepatitis B virus surface antigen", JOURNAL OF MEDICAL VIROLOGY, vol. 72, no. 2, 1 February 2004 (2004-02-01), pages 203-214, XP55362557, US ISSN: 0146-6615, DOI: 10.1002/jmv.10547
- S. T. TOH ET AL: "Deep sequencing of the hepatitis B virus in hepatocellular carcinoma patients reveals enriched integration events, structural alterations and sequence variations", CARCINOGENESIS., vol. 34, no. 4, 30 December 2012 (2012-12-30), pages 787-798, XP55362238, GB ISSN: 0143-3334, DOI: 10.1093/carcin/bgs406
- LIN, SELENA ET AL.: 'Detection of clonally expanded HBV DNA integration sites as a marker for early detection of HBV related HCC' CANCER RESEARCH vol. 73, no. SUPPL., 15 April 2013, XP008183295
- SU , YING-WEN ET AL.: '0uantitative analysis of plasma HBV DNA for early evaluation of the response to transcatheter arterial embolization for HBV- related hepatocellular carcinoma' WORLD JOURNAL OF GASTROENTEROLOGY vol. 11, no. 39, 2005, pages 6193 - 6196, XP055332544
- JIANG, SUZHEN ET AL.: 'Re-evaluation of the carcinogenic significance of hepatitis B virus integration in hepatocarcinogenesis' PLOS ONE vol. 7, no. ISSUE, 2012, pages 1 - 8, XP055332546
- MASON, WILLIAM S. ET AL.: 'Detection of clonally expanded hepatocytes in chimpanzees with chronic hepatitis B virus infection' JOURNAL OF VIROLOGY vol. 83, no. 17, 2009, pages 8396 - 8408, XP055332556
- SUNG, WING-KIN ET AL.: 'Genome-wide survey of recurrent HBV integration in hepatocellular carcinoma' NATURE GENETICS vol. 44, no. 7, 2012, pages 765 - 769, XP055197323
- LIN, SELENA ET AL.: 'Comprehensive analysis of the complexity of HBV DNA int egration sites in the circulation of patients with HBV related liver disease' PROCEEDINGS OF 2014 INTERNATIONAL SYMPOSIUM ON MOLECULAR MEDICINE AND INFECTIOUS DISEASE 16 June 2014, pages 67 - 68, XP008183337

## Description

### Cross Reference To Related Application

This application claims the benefit of U.S. Provisional Application No. 61/892796, filed October 18, 2013, the contents of which are adopted herein by reference.

### Technical Field

Aspects of the present disclosure relate generally to the field of using circulating nucleic acids in a subject as a biomarker to identify and monitor a disease development in the subject.

### Background

The fundamental cause of tumor/cancer has been attributed to genetic alterations caused by hereditary or environmental factors. These genetic alterations, once ir-repaired or irreparable, will accumulate and eventually cause normal cells to become malignant. As a tumor/cancer develops its own unique spectrum of genetic alterations, monitoring these alterations can provide information about the tumor/cancer.

Both normal and tumor/cancer cells undergo cycles of turnover where chromosomes of dead cells are fragmented and released into body fluids, such as blood circulation. Sequencing of these fragments indicates that these circulating cell-free DNA from the blood or serum of cancer patients carry the genetic alterations from the original tumor/cancer. This finding points to the potential of using circulating cell-free DNA.

The conventional design of using host genome sequences containing specific genetic alterations as probes for capturing cancer/tumor-specific nucleic acid sequences from total circulating cell-free DNA works for advanced cancer, where the tumor is sufficiently large and a significant amount of tumor-specific nucleic acid sequences (more than 5% of total circulating DNAs) is released into the circulation. Given its limited amount (0.01%∼1% in total blood), circulating cell-free DNA is hard to detect even in an advanced cancer. As a result, for early or intermediate stage of cancer, the proportion of circulating cancer/tumor DNA is too low to be reliably detected. Moreover, caner/tumor-specific mutations are usually single-base mutations, small insertions or deletions which are very difficult to be separated from nucleic acid sequences without such mutations released from the non-tumor somatic cells. In other words, not all circulating DNA bears the altered genetic information; most of the circulating DNA is unaltered and from host genome.

Conventional approach for cancer/tumor nucleic acid detection sampled from genomic DNA. Murakami et al. presented an approach to detect HBV DNA in liver, serum and peripheral blood mononuclear cells (Murakami, Y., Minami, M., Daimon, Y., & Okanoue, T. (2004). Hepatitis B virus DNA in liver, serum, and peripheral blood mononuclear cells after the clearance of serum hepatitis B virus surface antigen. Journal of medical virology, 72(2), 203-214.). In this approach, Alu-PCR were used to detect covalently closed circular HBV DNA, HBV core DNA, HBV S DNA, and HBV X DNA in genome DNA extracted from peripheral blood mononuclear cells and liver tissue. The product of Alu-PCR was sequenced to determine the presence of above targets. In this approach, known HBV DNA regions from patient's genome were amplified by PCR and sequenced.

For the ex-vivo detection of HBV DNA integration, Lin et al. presented an approach to detect HBV DNA integration sites in liver cancer cell lines (Lin, S., Jain, S., Block, T., Song, F., & Su, Y. H. (2013). Detection of clonally expanded HBV DNA integration sites as a marker for early detection of HBV related HCC.). Lin et al. disclosed a target enrichment assay and cloned DNA constructs to identify known HBV DNA integration sites in Hep3B cell line . The HBV DNA containing nucleotide position 1571 to 1960 were captured by biotinylated HBV RNA baits and cloned, and each of the clones were sequenced. In this approach, known HBV DNA integrations sites in Hep3B cell line were detected from cloning and sequencing.

### BRIEF DESCRIPTION OF THE DRAWINGS

Many aspects of the present disclosure can be better understood with reference to the following figures. The components in the figures are not necessarily drawn to scale with the emphasis instead being placed upon clearly illustrating the principles of the present disclosure.
FIG. 1 schematically shows general progression of virus-infected cells.
FIG. 2 schematically shows an exemplary method of obtaining target circulating cell-free DNA.
FIG. 3 shows the specificity of viral-host junction.
FIG. 4 shows the changes in the amount of specific viral-host junction before and after tumor resection.

### DETAILED DESCRIPTION

### 1. Introduction

Certain human tumors/cancers, such as hepatocellular carcinoma (HCC), are caused by chronic infection of hepatitis B virus (HBV). These cancers accumulate genetic alterations in their genomes. Among such alterations, a unique one is the integration of viral genome into the host genome, usually occurring in the early stage of infections. Superimposed upon these mutations are other somatic mutations that continue to occur and finally transform the cells to tumor/cancer.

As noted, when HCC cells turn over, fragmented genetic contents will be released into the body fluids. Circulating DNA which is DNA that floats freely in the circulatory system, such as blood, usually comprises DNA fragments. These fragments include those from host genome, from viral genome, and/or from the viral integration sites, such as the viral-host junction.

Infected cells, such as hepatocytes in a HBV-infected patient, proliferate if they become cancerous and so is the amount of the viral integrants carried by the infected cells. The amount of viral integrants thus is in proportion to the size of tumor/cancer in general. In addition, as the viral DNA integrates into the host genome at different sites, each tumor/cancer carries a unique spectrum of viral integration sites. This observation indicates that the viral integration sites, and/or the viral-host junction, are cancer/tumor-specific and can be used as biomarkers for the diagnosis of cancer/tumor development.

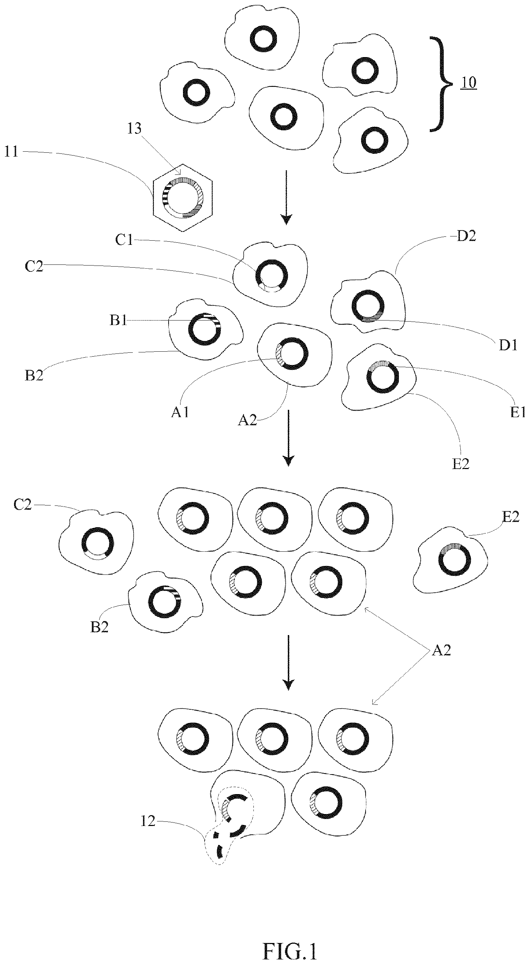

FIG. 1 shows a cancer development process of cells. Referring to FIG. 1, hepatocytes 10 in a subject generally have the same host genome. Referring again to FIG. 1, hepatocytes 10 comprise a plurality of hepatocytes A2, B2, C2, D2 and E2. Upon HBV 11 infection, HBV 11 can integrate its viral genome 13 into the host genome of the infected hepatocytes. Parts of HBV genome 13 are integrated into the host genome, generating infected hepatocytes with different viral integration sites and different integrated viral gene sequence. As show in FIG. 1, viral sequence A1 is integrated into cell A2, viral sequence B1 is integrated into cell B2, viral sequence C1 is integrated into cell C2, viral sequence D1 is integrated into cell D2 and viral sequence E1 is integrated into cell E2. The integration of HBV DNA sequences creates viral-host junctions in host cell genome. Infected cells A2, B2, C2, D2 and E2 grow, develop and accumulate additional genetic alterations with time. Both host and viral sequences, altered or not, might lead to proliferation, stable stage or cell death. Referring again to FIG. 1, cell A2 carries the alterations that induce malignant transformation and lead to proliferation or clonal expansion. It is to be noted that a viral-host junction may lead to malignant transformation or may be an insignificant integration that does not lead to proliferation.

Referring again to FIG. 1, infected cell A2 proliferates, expands in cell number and transforms into a malignant cell, which subsequently forms a cancerous or tumorous cell cluster. Cells B2, C2, D2, E2 do not go through malignant progression and remain in very small population or die out. All infected cells A2 bear the same hereditary information, including the host genome, at least partial viral genome, and the viral-host junctions. If the infected cells A2 proliferate, the number of cell A2-specific viral-host junctions would increase proportionally in general. The same viral-host junctions are present in the same cancerous cell lineage whether they trigger cancer development or not. As depicted schematically in FIG. 1, the cancerous clone goes through rapid proliferation and turnover, and some of the infected cells A2 rupture and die. DNA strands 12 of these ruptured and dead infected cells A2 are released into the circulatory system or body fluids such as blood. These DNA strands 12 become fragmented, float freely through the circulatory system and become part of circulating cell-free DNA (ctDNA) in the blood stream. As used herein, with reference to the present application, it shall be clearly understood that the terms "circulating cell-free DNA", "circulatory cell-free DNA" and "ctDNA" refer to DNA that is obtained from the blood stream or circulatory system of a subject or patient, wherein the DNA that is obtained from the blood stream or circulatory system of the subject or patient is either substantially free of other cellular components, or is essentially entirely free of other cellular components. Some of the ctDNA is later on digested or cleaned by functional cells such as macrophages while some remain in the blood stream especially when the ctDNA is in large amount. By examining and/or detecting the ctDNA in the circulatory system or body fluids, one can obtain information about cancer/tumor development.

### 2. Methods

Methods of performing the present invention are described below. It is to be noted that the methods, material and process described below are exemplary embodiments, and do not limit the scope of the invention in any way.

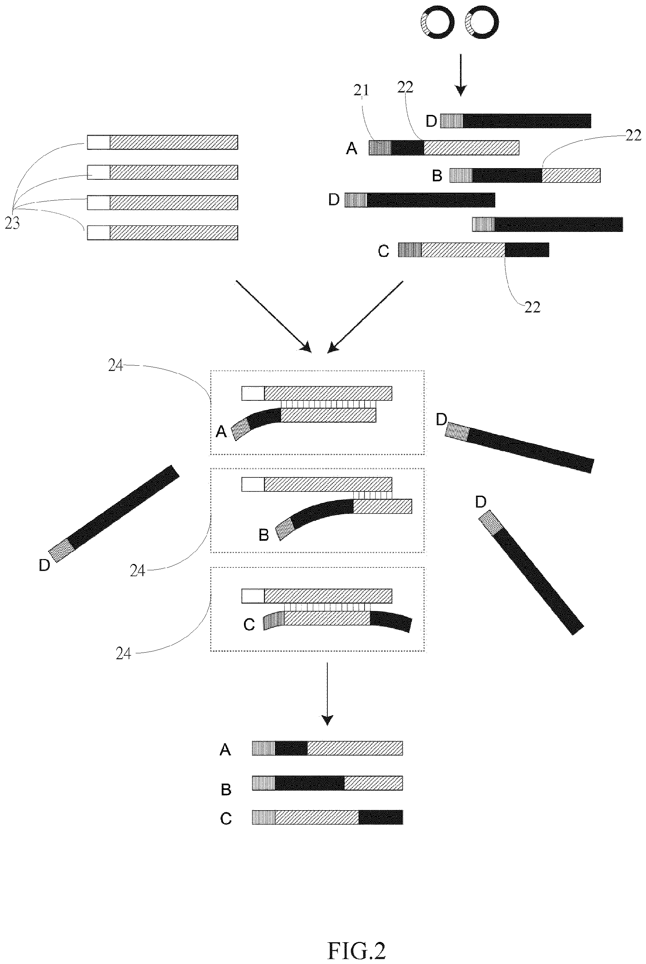

Referring to FIG. 2, a schematic view of isolating target ctDNA is shown. Circulating DNA from dead tumor/cancer cells is released into blood in fragments. Such ctDNA is collected and ligated with adaptors 21 and forms ctDNA A, B, C and D. The ctDNA is amplified by using any suitable approach, for instance, using a primer complementary to the sequence of the adaptor 21 in an appropriate amount. It is to be noted that preferred amplification methods amplify all ctDNA in a similar or the same proportions so that the amplified ctDNA provides genuine information as to the amount of ctDNA existing in the blood. In FIG. 2, sequences derived from viral genome are designated in hatch area while sequences derived from host genome are designated in black. The amplified ctDNA can be categorized into ctDNA having only host genome sequences (ctDNA D), ctDNAs having only viral genome sequence (not shown), and ctDNA having both viral and host genome sequences and thus comprising viral-host junctions 22 (ctDNA A, B, and C). According to a preferred approach, all ctDNA is incubated with polynucleotide probes 23 (derived from the viral genome sequence) to allow hybridization to occur. It is to be noted that the probes 23 shown in FIG. 2 may have different sequences even though all drawn alike. Referring again to FIG. 2, only ctDNA having viral genome sequence alone and ctDNA having at least a viral-host junction (ctDNA A, B and C) would form probe-ctDNA complexes 24. These complexes are isolated from the ctDNA that does not hybridize with the probe. The target ctDNA, the ctDNA having only viral genome sequence and ctDNA having at least a viral-host junction are then obtained from the complexes and separated. The sequences of target ctDNA are obtained. Tissue origins of the target ctDNA are identified based on tissue tropism and specificity of virus infection.

### 2.1 Subjects

Human subjects are employed in the tests to illustrate the present invention. Subject 1 has a 12x10x9 (cm) tumor diagnosed by computer tomography. According to the histological report when Subject 1 is employed in this test, Subject 1 is defined as a Grade III HCC patient. Subject 2 has a 18x13.5x9 (cm) tumor diagnosed by computer tomography. According to the histological report, Subject 2 is defined as a Grade III HCC patient. Subject 3 has s 8x7.5x7 (cm) tumor identified by computed tomography. According to the histological report, Subject 3 is defined as a Grade III HCC patient. Subject 4 has a 2x2x2 (cm) tumor and is at Grade II. Subject 5 has a tumor smaller than 2x2x2 (cm) and the stage of the cancer development is not determined and/or not available at the time of test enrollment.

### 2.2 Obtaining ctDNA in subjects

Multiple blood samples are obtained from each subject. Each time, blood is drawn, collected in a clinically suitable container and, if needed, stored in a suitable condition for later analysis. Each blood sample is processed to obtain serum, such as by centrifugation. ctDNA is extracted by a commercial kit, for example, MagNA Pure LC Total Nucleic acid Isolation kit (Roche). The tumor tissues are obtained and genomic DNAs of tumor cells are extracted.

### 2.3 Providing probes

Polynucleotides having HBV genome sequence are used as probes here. The probes can be either synthesized or obtained from the fragmentation of viral genome. Synthesis of the probes is described. Information of whole HBV genome sequences is obtained from the National Center for Biotechnology Information. Polynucleotides are synthesized according to the HBV genome sequence and cover the whole HBV genome sequence. The polynucleotides are synthesized using commercial kit, for example, Ion TargetSeq Custom Enrichment Kit (Life Technologies). All polynucleotides are about 50 to 200 or 50 to 120 residues in length. After the synthesis of probes, each probe is labeled, for example biotinylated, at least one end of the polynucleotide. Biotinylation of probes can be performed by using commercial kit, for example, Ion TargetSeq Custom Enrichment Kit (Life Technologies). The probes are subsequently attached or linked to a bead, for example through biotin.

### 2.4 Ligating adaptors

In order to proportionally amplify the ctDNAs obtained from the subject, certain DNA with known sequences are attached or ligated to at least one end or both ends of the ctDNA. Ligating adaptors to at least one end or both ends of the ctDNA can be performed by using TruSeq DNA Sample Preparation (Illumina), IonTorrent (Life Technologies), or other equivalent reagents.

### 2.5 Amplifying target ctDNAs

After the ctDNAs are ligated with adaptors, each ctDNA in the sample from the subject is amplified, for example by using TruSeq DNA Sample Preparation (Illumina) or IonTorrent (Life Technologies).

### 2.6 Capturing and isolating target ctDNAs

ctDNA samples of subjects are mixed with beads coated with biotinylated probes and incubated to allow hybridization between ctDNA and the probes. The ctDNA that have at least partial viral sequences anneal to the complementary sequences on the probes and form a bead-probe-ctDNA complex. The ctDNA that does not bind to the probes float freely and does not form any complex. The bead-probe-ctDNA complexes are separated from non-binding ctDNA by, for example, centrifugation. The complexes are obtained and target ctDNA is removed from the complexes and collected. Capturing of circulating DNA hybridized with the probes can be performed by using TargetSeq Hybridization & Wash Buffer Kit (Life Technologies), or by other equivalent reagents.

### 2.7 Sequencing and identifying target ctDNA

Primers having complementary sequences to the adaptor sequences are used to sequence the target ctDNAs. Target ctDNA is sequenced using IonTorrent platform, HiSeq 2500 (Illumina), or some other sequencing platforms.

### 3. Results

### 3.1 Target ctDNA sequences

### Subject 1

Table 1 shows top ten target sequences identified in the DNA samples obtained from Subject 1 tumor tissue. As shown, a junction sequence is inserted into the host chromosome (Host Chromosome #) at a specific integration position (Integration Position) with an accumulated read number (Accumulated Reads). Accumulated read number is obtained by sequencing result. Sequences having the same junction are counted to give the number of the junction present in the sample. Each sequence contains at least partial viral genome sequence (underlined) and partial host genome sequence and forms a viral-host junction.

**Table 1**

| Junction Data of Subject 1 Tumor | | | | |
|---|---|---|---|---|
| # | Host Chromosome # | Integration Position | Junction Sequence | Accumulated Reads |
| 1 | 17 | 22247083 | GGTCTTACATAAGAGGACTCAGAAAATACTTT GTGATGAT | 290 |
| 2 | 17 | 22251295 | AACTCCTTTTGAGAGCGCAGTGTTCGGTGCAG GTCCCCAG | 234 |
| 3 | 1 | 121360041 | ATCATCACAAAGTATTTTCTGAGTCCTCTTATG TAAGACC | 192 |
| 4 | 12 | 118876274 | TGAGGTGAGAGGATCTCTTGAGCACAGATGAT GGGATAGG | 115 |
| 5 | X | 58568585 | AAACGTCCACTTGCAGATTTTATGTAATTGGA AGTTGGGG | 102 |
| 6 | 8 | 56895765 | AGCAGGAAAATATATGCCCCACCTTCCCTTTC TCTGACCC | 106 |
| 7 | 1 | 121475300 | AGGAAGACTGCCTACTCCCACAGGCCTGAAAG CGCTCCAA | 85 |
| 8 | X | 58563641 | AGCATTCGGGCCAGGGTTCACTCAGGCTCAGG GCACATTG | 67 |
| 9 | 16 | 21525068 | GCATTTGGTGGTCTATAAGCACACCCGCCCAC ACCAATCT | 38 |
| 10 | 18 | 77932557 | CAAGACCAGCCTGAGGATGACTGTCTCTTAGA GGTGGAGA | 26 |

Table 2 shows the target sequences identified in the ctDNA samples obtained from the blood of Subject 1. ctDNA samples are obtained from Subject 1 13 days before a tumor excision. As shown, each sequence contains at least partial viral genome sequence (underlined) and partial host genome sequence and forms a viral-host junction.

**Table 2**

| Junction Data of Subject 1 Serum | | | | |
|---|---|---|---|---|
| # | Host Chromosome # | Integration Position | Junction Sequence | Accumulated Reads |
| 11 | 17 | 22251295 | CACTCCTTTTGAGAGCGCAGTGTTCAGGTGCA GGGTCCCC | 94 |
| 12 | 1 | 121360041 | ATCATCACAAAGTATTTTCTGAGTCCTCTTATG TAAGACC | 82 |
| 13 | 1 | 13727 | AACAGAAAGATTCGTCCCCAAATCCAATCTGT CTTCCATC | 68 |
| 14 | 8 | 56895765 | AGCAGGAAAATATATGCCCCACCTTCCCTTTC TCTGCCCT | 62 |
| 15 | 17 | 22247083 | GGTCTTACATAAGAGGACTCAGAAAATACTTT GTGATGAT | 42 |
| 16 | 16 | 21525068 | GCATTTGGTGGTCTATAAGCACACCCGCCCAC ACCAATCT | 31 |
| 17 | 8 | 56895953 | ATCATCCTGGGCTTTCTGCACTTCCCATAGGTA ATCAAAG | 16 |
| 18 | X | 58563641 | AGCATTCGGGCCAGGGTTCACTCAGGCTCAGG GCACATTG | 9 |

As illustrated in Tables 1 and 2, at least #3 (in tumor sample) and #12 (in serum sample), #1 and #15, and #2 and #11 each pair have the same viral-host junction sequences. Similar patterns (including the relative amount of reads) of viral-host junction sequences identified in both tumor DNA and ctDNA indicate that chimera ctDNA in serum is derived from tumor DNA. By selectively enriching the ctDNAs carrying at least a portion of the viral genome in the serum, viral-host junctions are identified to provide tumor-specific information about the subject.

### Subject 2

Table 3 shows the target sequences identified in the DNA samples obtained from Subject 2 tumor tissue. As shown, each sequence contains at least partial viral genome sequence (underlined) and partial host genome sequence and forms a viral-host junction.

**Table 3**

| Junction Data of Subject 2 Tumor | | | | |
|---|---|---|---|---|
| # | Host Chromosome # | Integration Position | Junction Sequence | Accumulated Reads |
| 1 | 3 | 111653312 | ATGAAGCTATTTATAATAAAACAAACTTTATTAAA TCTAGTTTAAATGCCTTACTCTCTTTTTTGCCTTCTG ACTTCTTTCCTTCTATTCGAGATCTCCT | 4183 |
| 2 | 2 | 80278757 | TTTCATTGTTGCTGTTTTTCAAATTGATTTTGGGAT CCAGCCTGTTATTCTACTCCCTTAACTTCATGGGAT ATGTAATTGGAAGTTGGGGTACTTTACC | 3772 |
| 3 | 3 | 111653206 | TCTCCCTTTAGACTTCAAACACTTCAAAATATGACT TCACTACAAAGCTTTATAGAATGCCAGCCTTCCAC AGAGTATGTAAATAATGCCTAGTTTTGAA | 1269 |
| 4 | 2 | 80278655 | CCAGCACATTTGTCTATAAATTTACATTCTTGGATA TTAGCAAAATTGCAAACAGACCAATTTATGCCTAC AGCCTCCTAGTACAAAGACCTTTAACCTA | 752 |
| 5 | 1 | 189879551 | TCCAGTGTTTGTGGGTTGAGCAGTATTATTGCATGG CCCAGTGGTGGTGGTTGATGTTCCTGGAAGTAGAG GACAAACGGGCAACATACCTTGGTAGTCC | 485 |
| 6 | 1 | 189879474 | TGCAAGTGGTTGCAGTTCTTTTGCTTTGCCACCACC ACTGGGCCATGCAAAACCTGCACGATTCCTGCTCA AGGAACCTCTATGTTTCCCTCTTGTTGCT | 174 |
| 7 | 20 | 60227034 | CAGGAGGAGGTGATGGACCCACTGGGTGGTGAAG AACAGTTTCTCTTCCAAAATTACTTCCCACCCAGGT GGCCAGATTCATCAACTCACCCCAACACAG | 169 |
| 8 | 22 | 26941239 | ATCTGTAAAATTGGGATCATCACACTTTCCTTTTAT TGGGGTTTAAATGAATACCCAAAGACAAAAGAAA ATTGGTAATAGAGGTAAAAAGGGACTCAAG | 100 |
| 9 | 20 | 60227112 | TGGCCGAGGCCATCTTCTAAATAAATGTGTGGAAG AGAAACTGTTCTTCAGTATTTGGTGTCTTTTGGAGT GTGGATTCGCACTCCTCCCGCTTACAGAC | 93 |
| 10 | 5 | 1295309 | AGGACGGGTGCCCGGGTCCCCAGTCCCTCCGCCAC GTGGGAAGCGCGGTCCAGACCAATTTATGCCTACA GCCTCCTAGTACAAAGACCTTTAACCTAAT | 37 |

Table 4 shows the target sequences identified in the ctDNA samples obtained from blood of Subject 2. Serum samples are obtained from Subject 2 at tumor excision. As shown, each sequence contains at least partial viral genome sequence (underlined) and partial host genome sequence and forms a viral-host junction.

**Table 4**

| Junction Data of Subject 2 Serum | | | | |
|---|---|---|---|---|
| # | Host Chromosome # | Integration Position | Junction Sequence | Accumulated Reads |
| 11 | 3 | 111653312 | ATGAAGCTATTTATAATAAAACAAACTTTATTAAATC TAGTTTAAATGCCTTACTCTCTTTTTTGCCTTCTGACT TCTTTCCTTCTATTCGAGATCTCCT | 3277 |
| 12 | 20 | 60227034 | CAGGAGGAGGTGATGGACCCACTGGTGGTGAAGA ACAGTTTCTCTTCCAAAATTACTTCCCACCCAGGTGG CCAGATTCATCAACTCACCCCAACACAG | 642 |
| 13 | 1 | 189879551 | TCCAGTGTTTGTGGGTTGAGCAGTATTATTGCATGGC CCAGTGGTGGTGGTTGATGTTCCTGGAAGTAGAGGA CAAACGGGCAACATACCTTGGTAGTCC | 373 |
| 14 | 2 | 50012582 | GTCCGTTGGTGGTGAACTGGGCAAGATAATTGCATG GCCCAGTGGTGGTGGTTGATGTTCCTGGAAGTAGAG GACAAACGGGCAACATACCTTGGTAGTC | 372 |
| 15 | 15 | 48344568 | AGATTGGTCTATAATTTTCTTTTACTATCTTCAGTATT TGGTATCTTTGGGAGTGTGGATTCGCACTCCTCCCGC TTACAGACCACCAAATGCCCCTATC | 237 |
| 16 | 2 | 80278757 | TTTCATTGTTGCTGTTTTTCAAATTGATTTTGGGATCC AGCCTGTTATTCTACTCCCTTAACTTCATGGGATATG TAATTGGAAGTTGGGGTACTTTACC | 230 |
| 17 | 20 | 60227112 | TGGCCGAGGCCATCTTCTAAATAAATGTGGAAGA GAAACTGTTCTTCAGTATTTGGTGTCTTTTGGAGTGT GGATTCGCACTCCTCCCGCTTACAGAC | 209 |
| 18 | 1 | 189879474 | TGCAAGTGGTTGCAGTTCTTTTGCTTTGCCACCACCA CTGGGCCATGCAAAACCTGCACGATTCCTGCTCAAG GAACCTCTATGTTTCCCTCTTGTTGCT | 205 |
| 19 | 2 | 50012660 | GTAAGCCATTGTGGCTTTCCTGACCAGCCCACCACCA CTGGGCCATGCAAAACCTGCACGATTCCTGCTCAAG GAACCTCTATGTTTCCCTCTTGTTGCT | 205 |
| 20 | 2 | 80278655 | CCAGCACATTTGTCTATAAATTTACATTCTTGGATAT TAGCAAAATTGCAAACAGACCAATTTATGCCTACAG CCTCCTAGTACAAAGACCTTTAACCTA | 64 |

As illustrated in Tables 3 and 4, at least #1 (in tumor sample) and #11 (in serum sample), #7 and #12, #5 and #3 both have the same viral-host junction sequences. Similar patterns of viral-host junction sequences identified in both tumor DNA and ctDNA show that chimera ctDNA in serum is derived from tumor DNA. By selectively enriching the target ctDNA in the serum, viral-host junctions are identified to provide tumor-specific information about the subject.

### Subject 3

Table 5 shows the target sequences identified in the DNA samples obtained from Subject 3 tumor tissue. As shown, each sequence contains at least partial viral genome sequence (underlined) and partial host genome sequence and forms a viral-host junction.

**Table 5**

| Junction Data of Subject 3 Tumor | | | | |
|---|---|---|---|---|
| # | Host Chromosome # | Integration Position | Junction Sequence | Accumulated Reads |
| 1 | 5 | 1295930 | GGAAATGGAGCCAGGCGCTCCTGCTGGCCGCGCAC CGGGCGCCTCACACCAGAACATCGCATCAGGACTC CTAGGACCCCTGCTCGTGTTACAGGCGGGG | 3024 |
| 2 | 8 | 111636420 | TCAAGCAGAAAAACCATGAAGATTTAAAAACTTGT AAATATTTGAATGTGGGCTCCACCCCAACAGTCCC CCGTGGGAGGGGTGAACCCTGGCCCGAAT | 635 |
| 3 | 14 | 52591737 | CTAAGGACACTACAGGAAACCAGCCCCGAAGTG ATTTCTTTTGAAATTCCAAATCTTTCTGTCCCCAAT CCCCTGGGATTCTTCCCCGATCATCAGTTG | 354 |
| 4 | 9 | 138857330 | CCTCGAAGCCTGTGCCAACCTAGCCCATTCCTCAG GCTCAGGGCCTCCTCACATCTGTGCCAGCAGCTCCT CCTCCTGCCTCCACCAATCGGCAGTCAGG | 190 |
| 5 | 1 | 68549419 | CATTGTTACTGTGATATGCTATAATTATTCTCACCT TATGTGTCCAAGGAATACTAACATTGAGATTCCCG AGATTGAGATCTTCTGCGACGCGGCGATT | 188 |
| 6 | 9 | 31455679 | ATGGAGAATACAGCACATTATTAGGAGTAAGTTTC CTTAAACACATTTTGATTTTTTGTACAATATGTTCC TGTGGCAATGTGCCCCAACTCCCAATTAC | 172 |
| 7 | 17 | 71434403 | TTTGCCACCTTCCTGCCACTTTGTAGATGCAAGATC TTGGGCAAGTTCCCGTGGGCGTTCACGGTGGTTTCC ATGCGACGTGCAGAGGTGAAGCGAAGTG | 138 |
| 8 | 12 | 126230889 | CAGTGGAAACAAAGCCACTGGGAAGTTCAAACTG AGAGAAGCCCACCACAAGTCTAGACTCTGTGGTAT TGTGAGGATTTTTGTCAACAAGAAAAACCCC | 135 |
| 9 | X | 35911295 | AGTATATCATCAGTTATTTTTCAAGGTTTTCTAAGT AAACAGTTTCTCAACCTTTACCCCGTTGCTCGGCAA CGGCCTGGTCTGTGCCAAGTGTTTGCTG | 124 |
| 10 | 10 | 75397400 | TCAGGGAGGGGATGTTGACTGCATTTTGGAGGTTC AGGGCCTACTAACAACTGTGCCAGCAGCTCCTCCT CCTGCCTCCACCAATCGGCAGTCAGGAAGG | 58 |

Table 6 shows the target sequences identified in the ctDNA samples obtained from blood of Subject 3. Serum samples are obtained from Subject 3 at tumor excision. As shown, each sequence contains at least partial viral genome sequence (underlined) and partial host genome sequence and forms a viral-host junction.

**Table 6**

| Junction Data of Subject 3 Serum | | | | |
|---|---|---|---|---|
| # | Host Chromosome # | Integration Position | Junction Sequence | Accumulated Reads |
| 11 | 5 | 1295930 | GGAAATGGAGCCAGGCGCTCCTGCTGGCCGCGCAC CGGGCGCCTCACACCAGAACATCGCATCAGGACTC CTAGGACCCCTGCTCGTGTTACAGGCGGGG | 153 |
| 12 | 8 | 111636420 | TCAAGCAGAAAAACCATGAAGATTTAAAAACTTGT AAATATTTGAATGTGGGCTCCACCCCAACAGTCCC CCGTGGGAGGGGTGAACCCTGGCCCGAAT | 52 |
| 13 | 21 | 47565536 | CCCGGGACCGACCCCAGGAAGAGCCAGGGCCCG GGTGATCCCTGCGGGGGTCTGGCTTTCAGTTATATG GATGATGTGGTATTGGGGGCCAAGTCTGTA | 27 |
| 14 | 21 | 28573066 | AATGAAAATCTCATTGATTTTTCACTTATAGGTTTT ACCTTAGAGCTCCTCCTCTGCCTAATCATCTCATGT TCATGTCCTACTGTTCAAGCCTCCAAGC | 25 |
| 15 | 7 | 87842849 | AGAATTGATACCTAAGCTGAGCAGAAATGAGGCCG ACCATGAAGTGAGTGCCTAATCATCTCATGTTCAT GTCCTACTGTTCAAGCCTCCAAGCTGTGCC | 24 |
| 16 | 7 | 148503201 | CGTAGGAAAGACAAGGTGGCATTGATGGAAAGCA GTAGTTTTTGAGCCCTTCGCAGACGAAGGTCTCAA TCGCCGCGTCGCAGAAGATCTCAATCTCGGG | 19 |
| 17 | 1 | 162277132 | TTAAAAAGGAGTTTTGTTTGTTAGTCTATTCACTCA TTTCAAGGAACATAGAAGAAGAACTCCCTCGCCTC GCAGACGAAGGTCTCAATCGCCGCGTCGC | 16 |
| 18 | 12 | 125048731 | CAGTTCCCTGGCTCCAAGCTCCCTCAAAAGATGCC CAGCTGGCCTTTCCCAAAGGCCTTGTAAGTTGGCG AGAAAGTAAAAGCCTGTTTTGCTTGTATAC | 15 |
| 19 | 7 | 30412226 | ACATGCCCTTCACTTCAGCCTGATGCTCCTGGCATA AGCTCAGCAATTTTGGAGTGCGAATCCACACTCCA AAAGACACCAAATATTCAAGAACAGTTTC | 13 |
| 20 | 13 | 84505952 | AATTTCCCCTGAATAGCTGCAGTACTCACAGACAC ACTGGATGCTACTCACCTCTGCCTAATCATCTCATG TTCATGTCCTACTGTTCAAGCCTCCAAGC | 13 |

As illustrated in Tables 5 and 6, similar patterns of viral-host junction sequences identified in both tumor DNA and ctDNA show that ctDNA in serum is derived from tumor DNA. By selectively enriching the target ctDNA in the serum, viral-host junctions are identified to provide tumor-specific information about the subject.

### 3.2 Tumor-specific viral-host junctions

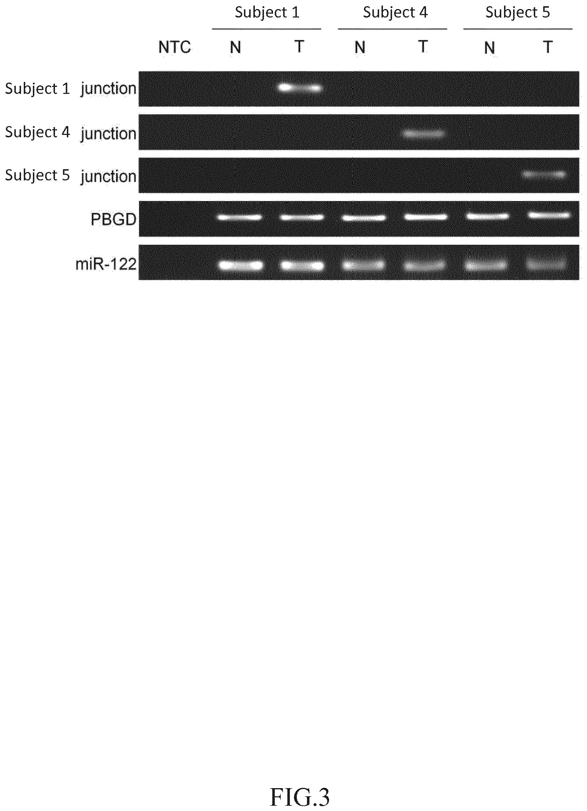

In FIG. 3, genomic DNA of Subject 1, Subject 4 and Subject 5 are processed and analyzed by polymerase chain reaction (PCR) and quantitative PCR. Genomic DNA (gDNA) from tumor tissues and non-tumor tissues is obtained. One chimera DNA sequence in tumor gDNA is identified and selected in each subject to serve as a marker to conduct the tests. Specifically, the chimera DNA sequence of Subject 1 used in this analysis is GGTCTTACATAAGAGGACTCAGAAAATACTTTGTGATGAT (viral genome sequence underlined), Subject 4 ACTTCAAAGACTGTGTGTTTCTAATTATTTTGGGGGACAT, and Subject 5 GTAGGCATAAATTGGTCTGTACCTCACTTCCCTGCTTTCC. The presence of the three specific viral-host junctions is determined in the tumor gDNA (T) and non-tumor gDNA (N). Porphobilinogen deaminase (PBGD) and miR-122 are used as internal control. No-template control (NTC) is also included. As illustrated in FIG. 3, the specific viral-host junction of Subject 1 is only present in tumor gDNA (T) but not in non-tumor gDNA (N). Same patterns are observed in Subject 4 and Subject 5, indicating that the identified viral-host junctions are tumor-specific and can be used as tumor-specific biomarkers.

### 3.3 Tumor development and viral-host junction amount

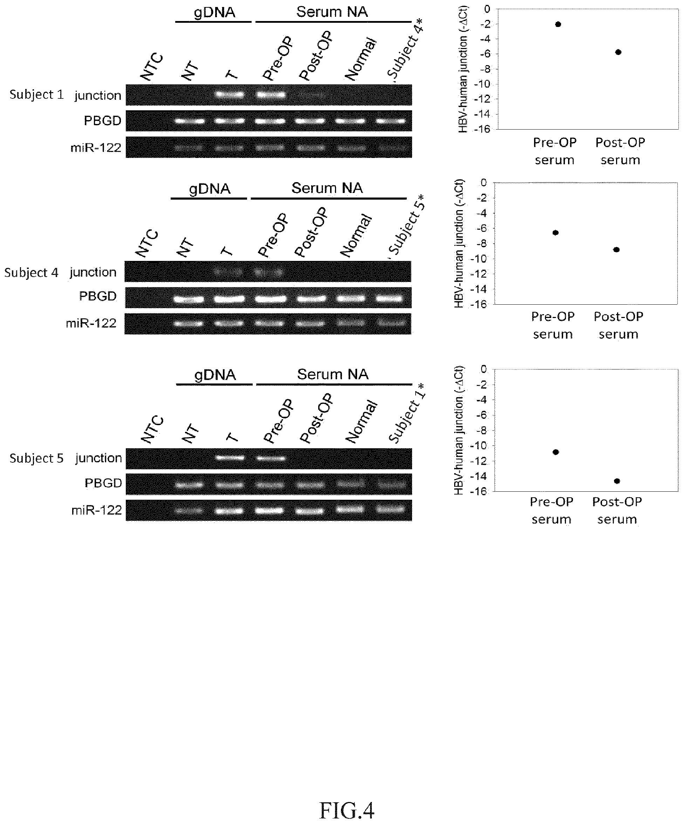

FIG. 4 shows the relationships between tumor size and the amount of specific viral-host junction sequence. Junction sequences used in FIG. 4 for each subject are the same as in FIG. 3. Serial blood samples of each subject are obtained at least at pre-operation and post-operation stages. Referring to FIG. 4, gDNA refers to genomic DNA, NTC refers to no-template control, NT refers to gDNA from non-tumor tissue, T refers to gDNA from tumor tissue, Serum NA refers to DNA obtained from serum, Pre-OP refers to serum DNA obtained at pre-operation stage, Post-OP refers to serum DNA obtained at post-operation stage, Subject 1* refers to serum DNA obtained from Subject 1 and is used in Subject 5 experiment, Subject 4* refers to serum DNA obtained from Subject 4 and is used in Subject 1 experiment, Subject 5* refers to serum DNA obtained from Subject 5 and is used in Subject 4 experiment and Normal refers to serum DNA obtained from a non-patient subject. Serum samples of Subject 1 are obtained at two time points, 13 days before tumor resection (operation) and 19 days after operation. Serum samples of Subject 4 are obtained 33 days before operation and 30 days after operation. Serum samples of Subject 5 are obtained 24 days before operation and 26 days after operation. Serum samples of non-patient subject (Normal) are also included as a control in FIG. 4. As shown in FIG. 4 left panel, the specific viral-host junction of each subject is only present in tumor gDNA and Pre-OP. In addition, the specific viral-host junction of Subject 1 is only present in Subject 1 DNA samples but not in Subject 4 DNA samples, suggesting that the viral-host junction identified is subject-specific. Referring now to the right panel of FIG. 4, the specific viral-host junction in the serum of Subject 1 is detected with relatively large amount in Pre-OP serum while the amount deceases sharply in Post-OP serum. The amount of the specific viral-host junction in Pre-OP serum and Post-OP serum is determined by qPCR and presented in the right panel of FIG. 4. In Subject 1, the amount of specific viral-host junction in Post-OP serum decreases by about 32-fold compared to in Pre-OP serum. Same patterns are observed in Subject 4 and Subject 5, showing that the viral-host junctions or the amount of junctions are tumor-specific, subject-specific, detectable in serum, reflective of tumor, and corresponsive to the tumor size changes, such as a decrease in size after an operation.

It is to be noted that by using the approach described in the present invention, mutated p53 or beta-catenin genes cannot be detected in the ctDNAs despite the mutations are identified in the tumor tissues (data not shown). The result shows that by using the method of present invention, tumor specific viral-host junctions (viral genome sequence insertion into host genome), and not conventional somatic mutations, are selectively enriched and obtained to provide cancer/tumor information.

The embodiments shown and described above are only examples. Even though numerous characteristics and advantages of the present technology have been set forth in the foregoing description, together with details of the structure and function of the present disclosure, the disclosure is illustrative only, and changes may be made in the detail, including in matters of shape, size and arrangement of the parts within the principles of the present disclosure up to, and including, the full extent established by the broad general meaning of the terms used in the claims.

## Claims

1. A method of identifying an amount of viral-host junctions in ctDNAs from a subject infected with hepatitis B virus comprising:
obtaining the ctDNAs in a serum obtained from the subject;
ligating each ctDNA with at least one adaptor;
amplifying all the ctDNAs ligating the at least one adaptor in a similar proportion by using a plurality of primers, wherein each of the primers is complementary to a sequence of the corresponding adaptor;
hybridizing polynucleotide probes with the ctDNAs ligated with the adaptors;
capturing and isolating target ctDNAs hybridized with the polynucleotide probes;
sequencing the target ctDNAs, wherein the target ctDNAs having one or more viral-host junction sequences, wherein each viral-host junction sequence comprises a viral genome sequence and a host genome sequence; and
accumulating a read number related to the viral-host junctions in the target ctDNAs to identify the amount of the viral-host junctions, wherein the amount of the viral-host junctions are defines as the accumulated read number, the read number is defined as the times the viral-host junction sequence appearing in the target ctDNAs, wherein the detected viral-host junctions sequences comprises same sequences, different sequences, or a combination thereof, and each viral-host junction sequence is counted to give the read number; and wherein the amount of the viral-host junctions are configured to indicate the hepatitis B virus-related tumor status.

2. The method of claim 1, further comprising comparing the detected the amount of the viral-host junctions of the subject at the different time points, to identify changes in size of the tumor.

3. The method of claim 1, wherein the target ctDNAs are enriched by the polynucleotide probes complementary to at least one sequence derived from hepatitis B viral genome.

4. The method of claim 1, wherein the polynucleotide probes cover the whole hepatitis B viral genome sequence.

5. The method of claim 1, wherein the ctDNAs ligating with the corresponding adaptor comprise one end of each ctDNA ligates with the corresponding adaptor, both ends of each ctDNA ligates with the corresponding adaptor, and a combination thereof.

6. A method of monitoring a tumor status in a subject caused by infection of hepatitis B virus, comprising:
obtaining the ctDNAs in a serum obtained from the subject;
ligating each ctDNA with at least one adaptor;
amplifying all the ctDNAs ligating the at least one adaptor in a similar proportion by using a plurality of primers, wherein each of the primers is complementary to a sequence of the corresponding adaptor;
hybridizing polynucleotide probes with the ctDNAs ligated with the adaptors;
capturing and isolating target ctDNAs hybridized with the polynucleotide probes;
sequencing the target ctDNAs, wherein the target ctDNAs having one or more viral-host junction sequences, wherein each viral-host junction sequence comprises a viral genome sequence and a host genome sequence;
accumulating a read number related to the viral-host junctions in the target ctDNAs to identify the amount of the viral-host junctions, wherein the amount of the viral-host junctions are defines as the accumulated read number, the read number is defined as the times the viral-host junction sequence appearing in the target ctDNAs, and wherein the detected viral-host junctions sequences comprises same sequences, different sequences, or a combination thereof, and each viral-host junction sequence is counted to give the read number; and
monitoring the tumor status based on the amount of the viral-host junctions in the target ctDNAs.

7. The method of claim 6, wherein the polynucleotide probes cover the whole hepatitis B viral genome sequence.

8. The method of claim 6, wherein the ctDNAs ligating with the corresponding adaptor comprise one end of each ctDNA ligates with the corresponding adaptor, both ends of each ctDNA ligates with the corresponding adaptor, and a combination thereof.

9. The method of claim 6, wherein the tumor is a hepatocelluar carcinoma induced by hepatitis B virus.

10. The method of claim 6, wherein the ctDNAs obtained at different time points, and wherein the different time points are selected from a cancerous condition, a pre-treatment condition, a post-treatment condition, a recurrence condition of the subject, and any combination thereof.

11. The method of claim 10, further comprising monitoring the tumor development of the subject by comparing the detected the amount of the viral-host junctions of the subject at the different time points.

12. The method of claim 10, wherein increases in the amount of the at least one viral-host junction in the ctDNAs of the subject are indicative of the tumor development from the post-treatment condition to a recurrence condition and decreases in the amount of the at least one viral-host junction in the ctDNAs of the subject are indicative of the tumor development from the pre-treatment condition to a post-treatment condition of the subject.

13. The method of claim 10, wherein increases in the amount of the at least one viral-host junction in the ctDNAs of the subject in the cancerous condition are indicative of growth of a tumor and decreases in the amount of the at least one viral-host junction in the circulating ctDNAs of the subject in the cancerous condition are indicative of shrinkage of the tumor.

14. A method of diagnosing a disease in a subject infected with hepatitis B virus, comprising:
detecting an amount of viral-host junctions in ctDNAs from the subject, comprising:
obtaining the ctDNAs in a serum obtained from the subject;
ligating each ctDNA with at least one adaptor;
amplifying all the ctDNAs ligating the at least one adaptor in a similar proportion by using a plurality of primers, wherein each of the primers is complementary to a sequence of the corresponding adaptor;
hybridizing polynucleotide probes with the ctDNAs ligated with the adaptors;
capturing and isolating target ctDNAs hybridized with the polynucleotide probes;
sequencing the target ctDNAs, wherein the target ctDNAs having one or more viral-host junction sequences, wherein each viral-host junction sequence comprises a viral genome sequence and a host genome sequence;
accumulating a read number related to the viral-host junctions in the target ctDNAs to identify the amount of the viral-host junctions, wherein the amount of the viral-host junctions are defines as the accumulated read number, the read number is defined as the times the viral-host junction sequence appearing in the target ctDNAs, and wherein the detected viral-host junctions sequences comprises same sequences, different sequences, or a combination thereof, and each viral-host junction sequence is counted to give the read number; and
diagnosing the disease in the subject infected with hepatitis B virus based on the amount of the viral-host junctions in the target ctDNAs.

15. The method of claim 14, wherein the disease is a cancer caused by chronic infection of hepatitis B virus.

## Patentansprüche

1. Verfahren zum Identifizieren einer Menge von Virus-Wirt-Verbindungen in ctDNAs eines mit Hepatitis B-Virus infizierten Subjekts, umfassend:
Erhalten der ctDNAs in einem Serum, das von dem Subjekt erhalten wurde;
Ligieren jeder ctDNA mit mindestens einem Adapter;
Amplifizieren aller ctDNAs, die den mindestens einen Adapter in einem ähnlichen Verhältnis unter Verwendung einer Vielzahl von Primern ligieren, wobei jeder der Primer komplementär zu einer Sequenz des entsprechenden Adapters ist;
Hybridisieren von Polynucleotidsonden mit den ctDNAs, die mit den Adaptern ligiert sind;
Einfangen und Isolieren von Target-ctDNAs, die mit den Polynucleotidsonden hybridisiert sind;
Sequenzieren der Target-ctDNAs, wobei die Target-ctDNAs eine oder mehrere Virus-Wirt-Verbindungssequenzen aufweisen, wobei jede Virus-Wirt-Verbindungssequenz eine Virusgenomsequenz und eine Wirtgenomsequenz umfasst; und
Akkumulieren einer Auslesungszahl bezüglich der Virus-Wirt-Verbindungen in den Target-ctDNAs zum Identifizieren der Menge der Virus-Wirt-Verbindungen, wobei die Menge der Virus-Wirt-Verbindungen als akkumulierte Auslesungszahl definiert ist, wobei die Auslesungszahl definiert ist als die Male, welche die Virus-Wirt-Verbindungssequenz in den Target-ctDNAs erscheint, wobei die detektierten Virus-Wirt-Verbindungen die gleichen Sequenzen, unterschiedliche Sequenzen oder eine Kombination davon umfassen und jede Virus-Wirt-Verbindungssequenz gezählt wird, um die Auslesungszahl zu ergeben; und wobei die Menge an Virus-Wirt-Verbindungen zum Anzeigen des Hepatitis B virenbezogenen Tumorstatus konfiguriert ist.

2. Verfahren nach Anspruch 1, weiter umfassend das Vergleichen der detektierten Menge an Virus-Wirt-Verbindungen des Subjekts zu unterschiedlichen Zeitpunkten, um Veränderungen in der Tumorgröße zu identifizieren.

3. Verfahren nach Anspruch 1, wobei die Target-ctDNAs durch die Polynucleotidsonden angereichert werden, die zu mindestens einer Sequenz komplementär sind, die von dem viralen Hepatitis B-Genom abgeleitet ist.

4. Verfahren nach Anspruch 1, wobei die Polynucleotidsonden die gesamte Hepatitis B-Virusgenomsequenz abdecken.

5. Verfahren nach Anspruch 1, wobei die ctDNAs, die mit dem entsprechenden Adapter ligieren, umfassen, dass ein Ende jeder ctDNA mit dem entsprechenden Adapter ligiert, beide Enden jeder ctDNA mit dem entsprechenden Adapter ligieren und eine Kombination davon.

6. Verfahren zur Überwachung eines Tumorstatus bei einem Subjekt, der durch eine Infektion mit dem Hepatitis B-Virus verursacht wird, umfassend:
Erhalten der ctDNAs in einem Serum, das von dem Subjekt erhalten wurde;
Ligieren jeder ctDNA mit mindestens einem Adapter;
Amplifizieren aller ctDNAs, die den mindestens einen Adapter in einem ähnlichen Verhältnis unter Verwendung einer Vielzahl von Primern ligieren, wobei jeder der Primer komplementär zu einer Sequenz des entsprechenden Adapters ist;
Hybridisieren von Polynucleotidsonden mit den ctDNAs, die mit den Adaptern ligiert sind;
Einfangen und Isolieren von Target-ctDNAs, die mit den Polynucleotidsonden hybridisiert sind;
Sequenzieren der Target-ctDNAs, wobei die Target-ctDNAs eine oder mehrere Virus-Wirt-Verbindungssequenzen aufweisen, wobei jede Virus-Wirt-Verbindungssequenz eine Virusgenomsequenz und eine Wirtsgenomsequenz umfasst;
Akkumulieren einer Auslesungszahl bezüglich der Virus-Wirt-Verbindungen in den Target-ctDNAs zum Identifizieren der Menge der Virus-Wirt-Verbindungen, wobei die Menge der Virus-Wirt-Verbindungen als akkumulierte Auslesungszahl definiert ist, wobei die Auslesungszahl definiert ist als die Male, welche die Virus-Wirt-Verbindungssequenz in den Target-ctDNAs erscheint, wobei die detektierten Virus-Wirt-Verbindungen die gleichen Sequenzen, unterschiedliche Sequenzen oder eine Kombination davon umfassen und jede Virus-Wirt-Verbindungssequenz gezählt wird, um die Auslesungszahl zu ergeben; und
Überwachen des Tumorstatus basierend auf der Menge der Virus-Wirt-Verbindungen in den Target-ctDNAs.

7. Verfahren nach Anspruch 6, wobei die Polynucleotidsonden die gesamte Hepatitis B-Virusgenomsequenz abdecken.

8. Verfahren nach Anspruch 6, wobei die ctDNAs, die mit dem entsprechenden Adapter ligieren, umfassen, dass ein Ende jeder ctDNA mit dem entsprechenden Adapter ligiert, beide Enden jeder ctDNA mit dem entsprechenden Adapter ligieren und eine Kombination davon.

9. Verfahren nach Anspruch 6, wobei der Tumor ein hepatozelluläres Karzinom ist, das durch Hepatitis B-Virus induziert wird.

10. Verfahren nach Anspruch 6, wobei die ctDNAs zu unterschiedlichen Zeitpunkten erhalten, und wobei die unterschiedlichen Zeitpunkte ausgewählt sind aus einem Krebszustand, einem Vorbehandlungszustand, einem Nachbehandlungszustand, einem Rekurrenzzustand des Subjekts und einer Kombination davon.

11. Verfahren nach Anspruch 10, weiter umfassend das Überwachen der Tumorentwicklung des Subjekts durch Vergleichen der detektierten Menge der Virus-Wirt-Verbindungen des Subjekts zu unterschiedlichen Zeitpunkten.

12. Verfahren nach Anspruch 10, wobei Zunahmen der Menge der mindestens einen Virus-Wirt-Verbindung in den ctDNAs des Subjekts für die Tumorentwicklung aus dem Nachbehandlungszustand zu einem Rekurrenzzustand indikativ sind und Abnahmen der Menge der mindestens einen Virus-Wirt-Verbindung in den ctDNAs des Subjekts für die Tumorentwicklung aus dem Vorbehandlungszustand zu einem Nachbehandlungszustand des Subjekts indikativ sind.

13. Verfahren nach Anspruch 10, wobei Zunahmen der Menge der mindestens einen Virus-Wirt-Verbindung in den ctDNAs des Subjekts im Krebszustand für das Wachstum eines Tumors indikativ sind und Abnahmen der Menge der mindestens einen Virus-Wirt-Verbindung in den zirkulierenden ctDNAs des Subjekts in dem Krebszustand für ein Schrumpfen des Tumors indikativ sind.

14. Verfahren zum Diagnostizieren einer Krankheit bei einem mit Hepatitis B-Virus infizierten Subjekt, umfassend:
Detektieren einer Menge von Virus-Wirt-Verbindungen in ctDNAs von dem Subjekt, umfassend:
Erhalten der ctDNAs in einem Serum, das von dem Patienten erhalten wurde;
Ligieren jeder ctDNA mit mindestens einem Adapter;
Amplifizieren aller ctDNAs, die den mindestens einen Adapter in einem ähnlichen Verhältnis unter Verwendung einer Vielzahl von Primern ligieren, wobei jeder der Primer komplementär zu einer Sequenz des entsprechenden Adapters ist;
Hybridisieren von Polynucleotidsonden mit den ctDNAs, die mit den Adaptern ligiert sind;
Einfangen und Isolieren von Target-ctDNAs, die mit den Polynucleotidsonden hybridisiert sind;
Sequenzieren der Target-ctDNAs, wobei die Target-ctDNAs eine oder mehrere Virus-Wirt-Verbindungssequenzen aufweisen, wobei jede Virus-Wirt-Verbindungssequenz eine Virusgenomsequenz und eine Wirtsgenomsequenz umfasst;
Akkumulieren einer Auslesungszahl bezüglich der Virus-Wirt-Verbindungen in den Target-ctDNAs zum Identifizieren der Menge der Virus-Wirt-Verbindungen, wobei die Menge der Virus-Wirt-Verbindungen als akkumulierte Auslesungszahl definiert ist, wobei die Auslesungszahl definiert ist als die Male, welche die Virus-Wirt-Verbindungssequenz in den Target-ctDNAs erscheint, und wobei die detektierten Virus-Wirt-Verbindungen die gleichen Sequenzen, unterschiedliche Sequenzen oder eine Kombination davon umfassen und jede Virus-Wirt-Verbindungssequenz gezählt wird, um die Auslesungszahl zu ergeben; und
Diagnostizieren der Krankheit bei mit Hepatitis B-Virus infizierten Subjekt basierend auf der Menge an Virus-Wirt-Verbindungen in den Target-ctDNAs.

15. Verfahren nach Anspruch 14, wobei die Krankheit ein Krebs ist, der durch die chronische Infektion mit Hepatitis B-Virus verursacht wird.

## Revendications

1. Procédé d'identification d'une quantité de jonctions virus-hôte dans des ADNtc provenant d'un sujet infecté par le virus de l'hépatite B comprenant :
l'obtention des ADNtc dans un sérum obtenu à partir du sujet ;
la ligature de chaque ADNtc avec au moins un adaptateur ;
l'amplification de tous les ADNtc ligaturant le au moins un adaptateur dans une proportion similaire en utilisant une pluralité d'amorces, dans lequel chacune des amorces est complémentaire d'une séquence de l'adaptateur correspondant ;
l'hybridation de sondes polynucléotidiques avec les ADNtc ligaturés avec les adaptateurs ;
la capture et l'isolement des ADNtc cibles hybridés avec les sondes polynucléotidiques ;
le séquençage des ADNtc, dans lequel les ADNtc cibles comportent une ou plusieurs séquences de jonctions virus-hôte, dans lequel chaque séquence de jonction virus-hôte comprend une séquence de génome viral et une séquence de génome de l'hôte ; et
le cumul d'un nombre de lectures en relation avec les jonctions virus-hôte dans les ADNtc cibles pour identifier la quantité de jonctions virus-hôte, dans lequel la quantité des jonctions virus-hôte est définie comme le nombre de lectures cumulées, le nombre de lectures est défini comme les fois où la séquence de jonction virus-hôte apparaît dans les ADNtc, dans lequel les séquences des jonctions virus-hôte détectées comprennent les mêmes séquences, des séquences différentes ou une combinaison de celles-ci, et chaque séquence de jonction virus-hôte est comptée pour donner le nombre de lectures; et dans lequel la quantité de jonctions virus-hôte est configurée pour indiquer l'état tumoral associé au virus de l'hépatite B.

2. Procédé selon la revendication 1, comprenant en outre la comparaison de la quantité détectée des jonctions virus-hôte du sujet aux points de temps différents, pour identifier les variations de taille de la tumeur.

3. Procédé selon la revendication 1, dans lequel les ADNtc cibles sont enrichis par les sondes polynucléotidiques complémentaires d'au moins une séquence dérivée du génome du virus de l'hépatite B.

4. Procédé selon la revendication 1, dans lequel les sondes polynucléotidiques couvrent la séquence entière du génome du virus de l'hépatite B.

5. Procédé selon la revendication 1, dans lequel les ADNtc se ligaturant avec l'adaptateur correspondant comprennent une extrémité de chaque ADNtc ligaturée avec l'adaptateur correspondant, les deux extrémités de chaque ADNtc ligaturées avec l'adaptateur correspondant, et une combinaison de celles-ci.

6. Procédé de surveillance d'un état tumoral chez un sujet provoqué par une infection par le virus de l'hépatite B, comprenant :
l'obtention des ADNtc dans un sérum obtenu à partir du sujet ;
la ligature de chaque ADNtc avec au moins un adaptateur ;
l'amplification de tous les ADNtc ligaturant le au moins un adaptateur dans une proportion similaire en utilisant une pluralité d'amorces, dans lequel chacune des amorces est complémentaire d'une séquence de l'adaptateur correspondant ;
l'hybridation de sondes polynucléotidiques avec les ADNtc ligaturés avec les adaptateurs ;
la capture et l'isolement des ADNtc cibles ligaturés avec les sondes polynucléotidiques ;
le séquençage des ADNtc cibles, dans lequel les ADNtc cibles comportent une ou plusieurs séquences de jonctions virus-hôte, dans lequel chaque séquence de jonction virus-hôte comprend une séquence du génome viral et une séquence du génome de l'hôte ;
le cumul d'un nombre de lectures en relation avec les jonctions virus-hôte dans les ADNtc cibles pour identifier la quantité de jonctions virus-hôte, dans lequel la quantité des jonctions virus-hôte est définie comme le nombre de lectures cumulées, le nombre de lectures est défini comme les fois où la séquence de jonction virus-hôte apparaît dans les ADNtc, et dans lequel les séquences des jonctions virus-hôte détectées comprennent les mêmes séquences, des séquences différentes ou une combinaison de celles-ci, et chaque séquence de jonction virus-hôte est comptée pour donner le nombre de lectures ; et
la surveillance de l'état tumoral sur la base de la quantité de jonctions virus-hôte dans les ADNtc cibles.

7. Procédé selon la revendication 6, dans lequel les sondes polynucléotidiques couvrent la séquence entière du génome du virus de l'hépatite B.

8. Procédé selon la revendication 6, dans lequel les ADNtc se ligaturant avec l'adaptateur correspondant comprennent une extrémité de chaque ADNtc ligaturée avec l'adaptateur correspondant, les deux extrémités de chaque ADNtc ligaturées avec l'adaptateur correspondant, et une combinaison de celles-ci.

9. Procédé selon la revendication 6, dans lequel la tumeur est un carcinome hépatocellulaire induit par le virus de l'hépatite B.

10. Procédé selon la revendication 6, dans lequel les ADNtc obtenus à différents points de temps, et dans lequel les différents points de temps sont sélectionnés parmi une condition cancéreuse, une condition prétraitement, une condition post-traitement, une condition de récurrence du sujet, et l'une quelconque de leurs combinaisons.

11. Procédé selon la revendication 10, comprenant en outre la surveillance du développement tumoral du sujet par comparaison de la quantité détectée des jonctions virus-hôte du sujet aux différents points de temps.

12. Procédé selon la revendication 10, dans lequel des augmentations de la quantité de l'au moins une jonction virus-hôte dans les ADNtc du sujet indiquent le développement tumoral de la condition post-traitement à une condition de récurrence et des diminutions de la quantité de l'au moins une jonction virus-hôte dans les ADNtc du sujet indiquent le développement tumoral de la condition prétraitement à une condition post-traitement du sujet.

13. Procédé selon la revendication 10, dans lequel des augmentations de la quantité de l'au moins une jonction virus-hôte dans les ADNtc du sujet dans la condition cancéreuse indiquent la croissance d'une tumeur et des diminutions de la quantité de l'au moins une jonction virus-hôte dans les ADNtc circulants du sujet dans la condition cancéreuse indiquent un rétrécissement de la tumeur.

14. Procédé de diagnostic d'une maladie chez un sujet infecté par le virus de l'hépatite B, comprenant :
la détection d'une quantité de jonctions virus-hôte dans des ADNtc provenant du sujet, comprenant :
l'obtention des ADNtc dans un sérum obtenu à partir du sujet ;
la ligature de chaque ADNtc avec au moins un adaptateur ;
l'amplification de tous les ADNtc ligaturant le au moins un adaptateur dans une proportion similaire en utilisant une pluralité d'amorces, dans lequel chacune des amorces est complémentaire d'une séquence de l'adaptateur correspondant ;
l'hybridation de sondes polynucléotidiques avec les ADNtc ligaturés avec les adaptateurs ;
la capture et l'isolement des ADNtc cibles ligaturés avec les sondes polynucléotidiques ;
le séquençage des ADNtc cibles, dans lequel les ADNtc cibles comportent une ou plusieurs séquences de jonctions virus-hôte, dans lequel chaque séquence de jonction virus-hôte comprend une séquence du génome viral et une séquence du génome de l'hôte ;
le cumul d'un nombre de lectures en relation avec les jonctions virus-hôte dans les ADNtc cibles pour identifier la quantité de jonctions virus-hôte, dans lequel la quantité des jonctions virus-hôte est définie comme le nombre de lectures cumulées, le nombre de lectures est défini comme les fois où la séquence de jonction virus-hôte apparaît dans les ADNtc, et dans lequel les séquences des jonctions virus-hôte détectées comprennent les mêmes séquences, des séquences différentes ou une combinaison de celles-ci, et chaque séquence de jonction virus-hôte est comptée pour donner le nombre de lectures ; et
le diagnostic de la maladie chez le sujet infecté par le virus de l'hépatite B sur la base de la quantité des jonctions virus-hôte dans les ADNtc cibles.

15. Procédé selon la revendication 14, dans lequel la maladie est un cancer provoqué par une infection chronique du virus de l'hépatite B.
